(19) Europäisches Patentamt
European Patent Office
Office européen des brevets

(11) **EP 3 635 735 B1**

(12) **EUROPÄISCHE PATENTSCHRIFT**

(45) Veröffentlichungstag und Bekanntmachung des Hinweises auf die Patenterteilung:
**20.09.2023   Patentblatt 2023/38**

(21) Anmeldenummer: **18807009.8**

(22) Anmeldetag: **13.11.2018**

(51) Internationale Patentklassifikation (IPC):
**G16H 30/40** *(2018.01)*       **G16H 50/20** *(2018.01)*
**A61B 5/11** *(2006.01)*

(52) Gemeinsame Patentklassifikation (CPC):
**G16H 30/40; A61B 5/1118; A61B 5/1128; G16H 50/20**

(86) Internationale Anmeldenummer:
**PCT/EP2018/081069**

(87) Internationale Veröffentlichungsnummer:
**WO 2019/096783 (23.05.2019 Gazette 2019/21)**

(54) **VERFAHREN, COMPUTERPROGRAMM UND VORRICHTUNG ZUR EINORDNUNG VON AKTIVITÄTEN EINES PATIENTEN**

METHOD, COMPUTER PROGRAM AND DEVICE FOR CLASSIFYING ACTIVITIES OF A PATIENT

PROCÉDÉ, PROGRAMME INFORMATIQUE ET DISPOSITIF DE CLASSIFICATION DES ACTIVITÉS D'UN PATIENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.11.2017   DE 102017010649**

(43) Veröffentlichungstag der Anmeldung:
**15.04.2020   Patentblatt 2020/16**

(73) Patentinhaber: **Drägerwerk AG & Co. KGaA
23558 Lübeck (DE)**

(72) Erfinder:
• **FRANZ, Frank
  23617 Stockelsdorf (DE)**
• **DIESEL, Jasper
  23564 Lübeck (DE)**

(56) Entgegenhaltungen:
**WO-A1-02/082999        DE-A1-102013 017 264
DE-A1-102015 013 031**

**Beschreibung**

Ausführungsbeispiele beziehen sich auf ein Verfahren, eine Vorrichtung und ein Computerprogramm zur Einordnung von Aktivitäten eines Patienten basierend auf Bilddaten des Patienten, insbesondere aber nicht ausschließlich, auf ein Konzept zur automatisierten Erkennung und Einordnung von intrinsischen und extrinsischen Patientenaktivitäten.

In der konventionellen Technik wird eine automatische Überwachung von Intensivpatienten primär durch Monitoring (Überwachen) von Vitalparametern durch Medizingeräte (z.B. hämodynamisches Monitoring, Beatmungsgerät) am Betten-/Patientenplatz durchgeführt. Die Medizingeräte sollen alarmieren, wenn ungewünschte physiologische Zustände eintreten. Nicht minder wichtig ist jedoch die Überwachung von Zustandsvariablen wie der Aktivität des Patienten.

Bisher findet eine solche Überwachung maßgeblich durch anwesendes pflegerisches Personal statt, das, je nach Zustand des Patienten, in mitunter sehr engmaschigen Zeitintervallen nach dem Patienten schauen sollte, was wiederum mit immensen Kosten verbunden ist.

Auf einer Intensivstation wird eine Vielzahl von Vitalparametern für die Patienten erhoben. Beispiele hierfür sind die Herzfrequenz, die Atemrate oder die Sauerstoffsättigung. Diese Vitalparameter ermöglichen eine verbesserte Einschätzung des Gesundheitszustandes des Patienten. Ein weiterer Vitalparameter, der bisher deutlich weniger Beachtung findet, ist die Aktivität des Patienten selbst. Insbesondere eine automatische Überwachung der Patientenaktivität ist nach heutigem Stand der Technik nur bedingt möglich. Dabei ist davon auszugehen, dass die ermittelte Patientenaktivität für eine Reihe von Anwendungen eine hohe Bedeutung hat. Beispiele hierfür sind die Aktivität als globaler Vitalparameter, die Erkennung von Aufwachereignissen, die Beurteilung der Sedierungstiefe oder die Beurteilung der Bewegungsfähigkeit einzelner Körperteile.

Die meisten bekannten Verfahren und Vorrichtungen bedienen sich Drucksensoren im Kontakt mit dem Patienten selbst oder zumindest dessen Liegefläche oder Beschleunigungssensoren, die direkt am Patienten befestigt sind.

Beispielsweise sind drucksensorbasierte Systeme bekannt, die für eine Vielzahl von Aufgaben Verwendung finden sollen. Ein solches System zeichnet sich durch Drucksensoren aus, welche unter der Matratze an einem Patientenbett angebracht sind. Bewegungen der auf der Matratze liegenden Person werden aufgezeichnet und ausgewertet. Druckbasierte Systeme sind direkt in Patientennähe angebracht und können somit Beeinträchtigungen hinsichtlich Reinigung und Hygiene mit sich bringen. Darüber hinaus sind solche Sensoren statisch mit der Matratze des Patienten verbunden und verfügen daher auch über einen statischen Erfassungsbereich.

Die Veröffentlichung "Implementation of a Real-Time Human Movement Classifier Using a Triaxial Acceleromater for Ambulatory Monitoring" von Karantonis et al aus dem Jahre 2006 beschreibt ein System, das mit einem an der Hüfte eines Patienten befestigten Beschleunigungssensor in der Lage sein soll, Perioden von Aktivität und Ruhe, sowie Events wie Gehen und Fallen zu erkennen. Direkt am Patienten befestigte Sensoren überwachen jeweils den Patientenbereich, an dem sie befestigt sind.

In der Patentanmeldung LU90722 wird ein Verfahren beschrieben, um einen Patienten in einem Krankenbett mittels Kameras zu überwachen. Dabei werden "Situationsbilder" aufgenommen, die dann über die Zeit miteinander verglichen werden, um Veränderungen zu detektieren. Überschreiten die Veränderungen einen Schwellwert, wird eine Aktion ausgelöst. Weiterhin wird erläutert, dass in den Situationsbildern Kanten und/oder Konturen erkannt werden könnten, wodurch sich nicht relevante Körperteile ausschließen ließen. Es wird ebenso erwähnt, dass man durch eine Objekterkennung Daten erhalten könne, die die Haltung des Patienten beschrieben, sowie Rückschlüsse auf Bewegungen einzelner, verletzter Körperteile zuließen.

Die Patentanmeldung WO02082999 beschäftigt sich mit der Erkennung von Krampfanfällen mit der Hilfe von Sequenzen von Bildern. Dazu wird eine Interessenzone festgelegt, das Bild dieser Interessenzone in kleinere Regionen unterteilt und für jede Region der Grad der Veränderung über die Zeit quantisiert. Anschließend versucht ein Prozessor periodische Bewegungen zu erkennen, um so auf Krampfanfälle zu schließen.

Die Druckschrift DE 10 2013 017 264 A1 offenbart ein Verfahren für die Überwachung eines Patienten innerhalb eines medizinischen Überwachungsbereichs mittels eines Überwachungssystems mit einer Tiefenkameravorrichtung, aufweisend die folgenden Schritte: Erzeugen einer Punktewolke des Überwachungsbereichs mit dem Überwachungssystem, Auswerten der Punktewolke zur Erkennung vordefinierter Objekte, insbesondere Personen, Bestimmen des Ortes wenigstens eines erkannten Objekts im Überwachungsbereich, Vergleich des bestimmten Ortes des wenigstens einen erkannten Objekts mit wenigstens einem Vorgabewert für den Ort dieses erkannten Objekts.

Weitere Details finden sich in

- Achilles, F. (2016), Patient MoCap: "Human Pose Estimation Under Blanket Occlusion for Hospital Monitoring Applications", International Conference on Medical Image Computing and Computer-Assisted Intervention,
- Barnich, O., & Van Droogenbroeck, M. (2011), ViBe: "A universal background subtraction algorithm for video sequences", Image Processing, IEEE Transactions on, und
- Girshick, R., Donahue, J., Darrell, T., & Malik, J. (2014), "Rich feature hierarchies for accurate object detection and semantic segmentation", CVPR (computer Vision and Pattern Recognition).

Es besteht daher ein Bedarf ein verbessertes Konzept für die Erkennung und Einordnung von Patientenaktivitäten zu schaffen. Diesem Bedarf werden ein erfindungsgemäßes computerimplementiertes Verfahren, eine erfindungsgemäße Vorrichtung und ein erfindungsgemäßes Computerprogramms gemäß den anhängigen unabhängigen Ansprüchen gerecht, welche die Erfindung definieren.

Ausführungsbeispiele der vorliegenden Erfindung basieren auf der Erkenntnis, dass kamerabasierte Überwachungssysteme für Intensivstationen verwendet werden können, um eine Aktivität eines Patienten zu überwachen. Dabei können basierend auf erfassten Bilddaten des Patienten beispielsweise automatisiert Ereignisse (auch engl. "Events") generiert werden, die über ein Kommunikationsnetzwerk an geeignete Abnehmer weitergeleitet werden. Die Events können dabei helfen die Arbeitskräfte zu entlasten, die Patientensicherheit erhöhen und eine Verbesserung der Pflege bewirken.

Ausführungsbeispiele stellen ein Verfahren, ein Computerprogramm und eine Vorrichtung zur Bestimmung einer Aktivität in zweidimensionalen Bilddaten und/oder in dreidimensionalen Punktewolken bereit. Ein Anwendungsfall ist das Bestimmen von Aktivität eines Patienten (vorrangig auf einer Intensivstation, aber z.B. auch in Pflegeheimen etc.). Erfindungsgemäß wird für die Aktivität zwischen intrinsischer (aktiver) und extrinsischer (passiver) Aktivität unterschieden. Es ist ein weiterer Kerngedanke von Ausführungsbeispielen aktive und passive Aktivitäten eines Patienten zu unterscheiden, bzw. allgemein hinsichtlich Patientenaktivitäten zumindest zwischen aktiven und passiven Aktivitäten zu unterscheiden. Die erstere (aktiv hervorgerufene) Aktivität kennzeichnet sich dadurch, dass diese vom Patienten selbst induziert ist oder hervorgerufen wird. Die letztere passive Aktivität ist hingegen auf äußere Einflüsse zurückzuführen.

Auf einer Intensivstation ist der Patient in der Regel von der Außenwelt nicht völlig abgeschottet. Pflegepersonal und Besucher interagieren mit dem Patienten körperlich und Geräte können zur Bewegung des Patienten führen, ohne dass dieser selbst für die Bewegung verantwortlich war. Diese von außen induzierte Aktivität des Patienten kann Messergebnisse alternativer, nicht differenzierender Verfahren wie Sensormatratzen oder Inertialsensorik verfälschen.

Erfindungsgemäß ist ein computerimplementiertes Verfahren zur Einordnung von Aktivitäten eines Patienten basierend auf Bilddaten des Patienten in einer Patientenlagerungsvorrichtung bereitgestellt.

Das Verfahren umfasst ein Detektieren einer Aktivität des Patienten basierend auf den Bilddaten. Das Verfahren umfasst darüber hinaus ein Bestimmen von Einordnungsinformation für die Aktivität aus den Bilddaten. Die Einordnungsinformation umfasst zumindest Information darüber, ob die detektierte Aktivität des Patienten aktiv vom Patienten oder passiv durch einen äußeren Einfluss hervorgerufen wurde. Das Verfahren umfasst ferner ein Bereitstellen von Information über die Aktivität und der Einordnungsinformation. Ausführungsbeispiele können so eine robuste Erkennung und Einordnung von Aktivitäten für Personen in Patientenlagerungsvorrichtungen erlauben.

In manchen Ausführungsbeispielen kann das Verfahren ferner ein Erfassen der Bilddaten in der Umgebung des Patienten und der Patientenlagerungsvorrichtung mit mehreren Sensoren vorsehen. Die Nutzung mehrerer Sensoren kann dabei zu einer robusteren Erkennung und Einordnung der Patientenaktivitäten führen.

Die Erfindung basiert ferner auf dem Gedanken, eine Interessenzone innerhalb der Bilddaten zu betrachten. Manche Ausführungsbeispiele können daher eine Erkennung von Aktivitäten auf Interessenzonen (z.B. "Bett") einschränken. Dementsprechend erfolgt ein Bestimmen einer Interessenzone in den Bilddaten, wobei das Detektieren der Aktivität in der Interessenzone erfolgt. Dabei ist ferner eine Bestimmung einer weiteren Zone vorgesehen, die die Interessenzone zumindest teilweise umschließt. Das Bestimmen der Einordnungsinformation kann dann ferner eine Bestimmung, ob eine Aktivität innerhalb der Interessenzone zu einer Aktivität in der weiteren Zone korrespondiert, umfassen.

In einigen weiteren Ausführungsbeispielen kann das Bereitstellen der Information über die Aktivität einen zugeordneten Zeitstempel umfassen. Dieser Zeitstempel kann beispielsweise zu Dokumentationszwecken verwendet werden oder auch zum zeitlichen Abgleich von Aktivitäten in den verschiedenen Zonen. Das Bestimmen der Einordungsinformation kann in einigen Ausführungsbeispielen ein Überprüfen vorhandener Aktivitäten anhand des Zeitstempels umfassen. Ausführungsbeispiele können daher eine Überprüfung nach einer zeitlichen Übereinstimmung oder Zuordnung der Aktivitäten in den Zonen umfassen.

Die detektierte Aktivität des Patienten kann dann entsprechend als aktiv vom Patienten hervorgerufen eingeordnet werden, wenn die detektierte Aktivität innerhalb der Interessenzone zu keiner Aktivität in der weiteren Zone korrespondiert. Die detektierte Aktivität des Patienten kann als passiv durch äußeren Einfluss hervorgerufen eingeordnet werden, wenn die detektierte Aktivität innerhalb der Interessenzone zu einer Aktivität in der weiteren Zone korrespondiert. Die Interessenzone und die weitere Zone können so eine Unterscheidung der Aktivitäten zumindest nach aktiv und passiv erlauben, so wie eine Fokussierung der Erkennung auf ein Interessenzone, beispielsweise bestimmte Gliedmaßen, Körperregionen, oder der ganze Patient.

In weiteren Ausführungsbeispielen können die Zonen auch nachgeführt werden. Das Verfahren kann demnach ein Nachführen der Interessenzone basierend auf den Bilddaten umfassen. In anderen Worten können die Interessenzone und/oder die weitere Zone aufgrund einer Analyse der Bilddaten einem zu überwachenden Körperteil oder Bereich des Patienten adaptiv nachgeführt werden. Dies kann beispielsweise auch durch Konfigurationsänderungen der Patientenlagerungsvorrichtung hervorgerufen werden. Ausführungsbeispiele können so eine manuelle Nachführung oder Unschärfen beim zu überwachenden Bereich reduzieren oder ganz vermeiden.

In einigen weiteren Ausführungsbeispielen kann das Verfahren ein Detektieren von zumindest einer weiteren Person in der Umgebung des Patienten mittels der Bilddaten und ein Detektieren von Interaktionen zwischen der zumindest einen weiteren Person und dem Patienten umfassen. Basierend auf der Interaktion kann dann eine Bestimmung der Einordnungsinformation erfolgen. Ausführungsbeispiele können so Information über weitere Personen in der Szene bzw. über deren Aktivitäten mit in die Erkennung und Einordnung der Patientenaktivität einfließen lassen. In manchen Ausführungsbeispielen kann auch eine Detektion einer Konfigurationsveränderung der Patientenlagerungsvorrichtung basierend auf den Bilddaten oder auch basierend auf anderen Informationen, wie beispielsweise Stellgradrückmeldungen der Patientenlagerungsvorrichtung, bei der Bestimmung der Einordnungsinformation berücksichtigt werden. Beispielsweise können in einigen Ausführungsbeispielen auch mehrere Interessenzonen mit zugehörigen weiteren Zonen definiert sein, um so zwischen verschiedenen Aktivitäten weiter zu unterscheiden, z.B. Erkennen einer Aktivität in der Augenpartie um einen Aufwachvorgang zu detektieren, Überwachen des ganzen Patienten in der Patientenlagerungsvorrichtung, Überwachen einzelner Gliedmaßen, Überwachen von Körperbereichen zur Detektion von Lähmungen (Hemiplegia, Paraplegia), usw.

In manchen Ausführungsbeispielen kann auch eine Detektion eines Mobilisierungsgerätes basierend auf den Bilddaten erfolgen. Die Einordungsinformation kann dann auch basierend auf dem Vorhandensein eines Mobilisierungsgerätes erfolgen. Mögliche Mobilisierungsgeräte können dabei beispielsweise Bettfahrräder, Bewegungsschienen, eine Patientenlagerungsvorrichtung selbst usw sein. In einigen weiteren Ausführungsbeispielen kann ein Aktivitätenprofil bestimmt werden, das Information über einen zeitlichen Verlauf von aktiv oder passiv hervorgerufenen Aktivitäten des Patienten umfasst. Damit können Ausführungsbeispiele über die Profile diagnostische Hilfsmittel oder Mittel zur Beurteilung eines Genesungsprozesses bereitstellen.

In weiteren Ausführungsbeispielen können Verfahren des "Deep Learning" eingesetzt werden, um die Bilddaten zu verarbeiten. Das Bestimmen der Einordnungsinformation kann dann ein Verarbeiten der Bilddaten mit Verfahren des "Deep Learning" umfassen. Die Verwendung von Deep Learning kann Vorteile bei der Erkennung und Einordnung neuer Aktivitäten haben.

Ausführungsbeispiele schaffen darüber hinaus eine Vorrichtung mit einer Recheneinrichtung, die ausgebildet ist, um ein Verfahren gemäß der obigen Beschreibung durchzuführen. Ausführungsbeispiele schaffen auch ein Computerprogramm mit einem Programmcode zur Durchführung eines der Verfahren gemäß der obigen Beschreibung, wenn der Programmcode auf einem Computer, einem Prozessor oder einer programmierbaren Hardwarekomponente ausgeführt wird.

Weitere vorteilhafte Ausgestaltungen werden nachfolgend anhand der in den Zeichnungen dargestellten Ausführungsbeispiele, auf welche Ausführungsbeispiele generell jedoch nicht insgesamt beschränkt sind, näher beschrieben. Es zeigen:

Fig. 1 ein Blockdiagramm eines Ausführungsbeispiels eines Verfahrens zur Einordnung von Aktivitäten eines Patienten;

Fig. 2 einen Patienten und eine Patientenlagerungsvorrichtung in einem Ausführungsbeispiel;

Fig. 3 ein Flussdiagramm eines weiteren Ausführungsbeispiels,

Fig. 4 ein Blockdiagramm eines weiteren Ausführungsbeispiels eines Verfahrens zur Nachführung einer Interessenzone;

Fig. 5 eine Illustration zur Veranschaulichung einer Interessenzone und einer weiteren Zone in einem Ausführungsbeispiel;

Fig. 6 ein Blockdiagramm eines weiteren Ausführungsbeispiels eines Verfahrens zur Unterscheidung zwischen intrinsischer und extrinsischer Aktivität eines Patienten; und

Fig. 7 eine Veranschaulichung zur Weiterverarbeitung von als intrinsisch und extrinsisch markierten Aktivitäten in einem Ausführungsbeispiel.

Verschiedene Ausführungsbeispiele werden nun ausführlicher unter Bezugnahme auf die beiliegenden Zeichnungen beschrieben, in denen einige Ausführungsbeispiele dargestellt sind.

Bei der nachfolgenden Beschreibung der beigefügten Figuren, die lediglich einige exemplarische Ausführungsbeispiele zeigen, können gleiche Bezugszeichen gleiche oder vergleichbare Komponenten bezeichnen. Ferner können zusammenfassende Bezugszeichen für Komponenten und Objekte verwendet werden, die mehrfach in einem Ausführungsbeispiel oder in einer Zeichnung auftreten, jedoch hinsichtlich eines oder mehrerer Merkmale gemeinsam beschrieben werden. Komponenten oder Objekte, die mit gleichen oder zusammenfassenden Bezugszeichen beschrieben werden, können hinsichtlich einzelner, mehrerer oder aller Merkmale, beispielsweise ihrer Dimensionierungen, gleich, jedoch gegebenenfalls auch unterschiedlich ausgeführt sein, sofern sich aus der Beschreibung nicht etwas anderes explizit oder implizit ergibt. Optionale Komponenten sind in den Figuren mit gestrichelten Linien oder Pfeilen dargestellt.

Obwohl Ausführungsbeispiele auf verschiedene Weise modifiziert und abgeändert werden können, sind Ausführungsbeispiele in den Figuren als Beispiele dargestellt und werden hierin ausführlich beschrieben. Es sei jedoch klargestellt, dass nicht beabsichtigt ist, Ausführungsbeispiele auf die jeweils offenbarten Formen zu beschränken, sondern dass Ausführungsbeispiele vielmehr sämtliche funktionale und/oder strukturelle Modifikationen, Äquivalente und Alternativen, die im Bereich der Erfindung liegen, abdecken sollen. Gleiche Bezugszeichen bezeichnen in der gesamten Figurenbeschreibung gleiche oder ähnliche Elemente.

Man beachte, dass ein Element, das als mit einem anderen Element "verbunden" oder "verkoppelt" bezeichnet wird, mit dem anderen Element direkt verbunden oder verkoppelt sein kann oder dass dazwischenliegende Elemente vorhanden sein können. Wenn ein Element dagegen als "direkt verbunden" oder "direkt verkoppelt" mit einem anderen Element bezeichnet wird, sind keine dazwischenliegenden Elemente vorhanden. Andere Begriffe, die verwendet werden, um die Beziehung zwischen Elementen zu beschreiben, sollten auf ähnliche Weise interpretiert werden (z.B., "zwischen" gegenüber "direkt dazwischen", "angrenzend" gegenüber "direkt angrenzend" usw.).

Die Terminologie, die hierin verwendet wird, dient nur der Beschreibung bestimmter Ausführungsbeispiele und soll die Ausführungsbeispiele nicht beschränken. Wie hierin verwendet, sollen die Singularformen " einer", "eine", "eines" und "der, die, das" auch die Pluralformen beinhalten, solange der Kontext nicht eindeutig etwas anderes angibt. Ferner sei klargestellt, dass die Ausdrücke wie z.B. "beinhaltet", "beinhaltend", "aufweist", "umfasst", "umfassend" und/oder "aufweisend", wie hierin verwendet, das Vorhandensein von genannten Merkmalen, ganzen Zahlen, Schritten, Arbeitsabläufen, Elementen und/oder Komponenten angeben, aber das Vorhandensein oder die Hinzufügung von einem bzw. einer oder mehreren Merkmalen, ganzen Zahlen, Schritten, Arbeitsabläufen, Elementen, Komponenten und/oder Gruppen davon nicht ausschließen.

Solange nichts anderes definiert ist, haben sämtliche hierin verwendeten Begriffe (einschließlich von technischen und wissenschaftlichen Begriffen) die gleiche Bedeutung, die ihnen ein Durchschnittsfachmann auf dem Gebiet, zu dem die Ausführungsbeispiele gehören, beimisst. Ferner sei klargestellt, dass Ausdrücke, z.B. diejenigen, die in allgemein verwendeten Wörterbüchern definiert sind, so zu interpretieren sind, als hätten sie die Bedeutung, die mit ihrer Bedeutung im Kontext der einschlägigen Technik konsistent ist, und nicht in einem idealisierten oder übermäßig formalen Sinn zu interpretieren sind, solange dies hierin nicht ausdrücklich definiert ist.

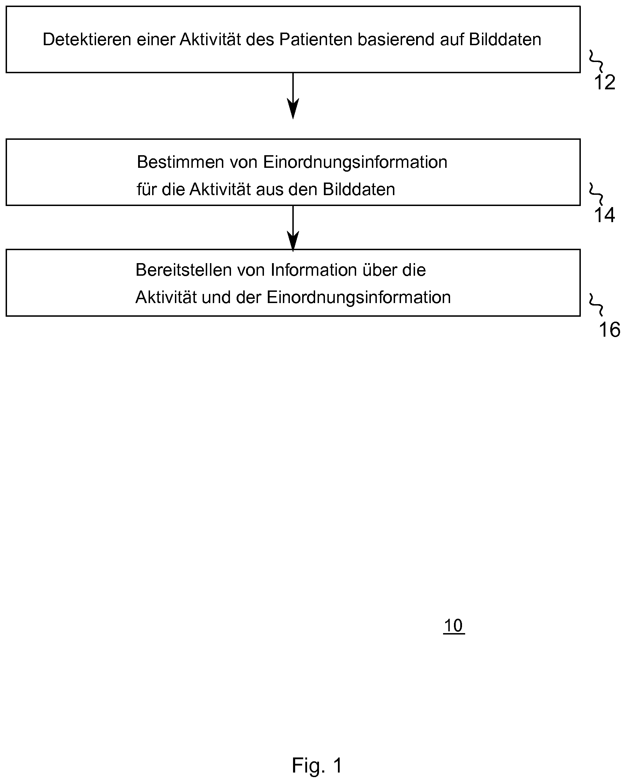

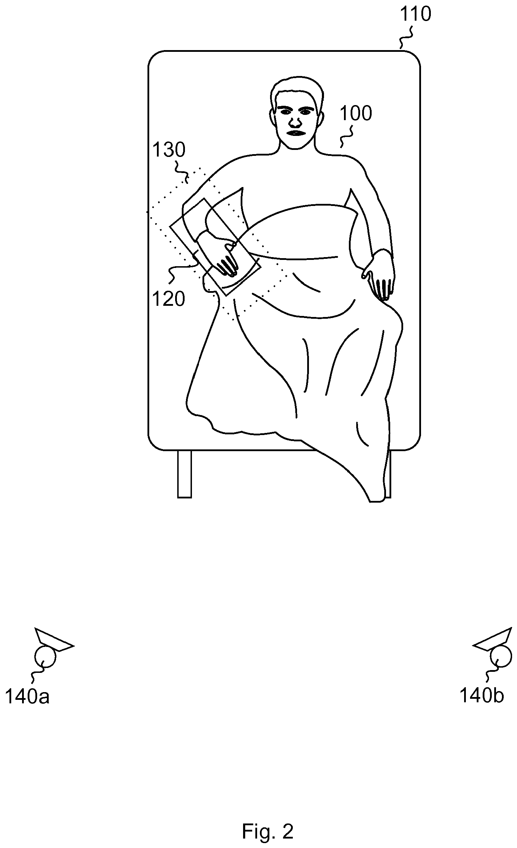

Fig. 1 zeigt in einem Blockdiagramm ein Ausführungsbeispiel eines Verfahrens 10 zur Einordnung von Aktivitäten eines Patienten 100 basierend auf Bilddaten des Patienten 100 in einer Patientenlagerungsvorrichtung 110, wobei die Fig. 2 den Patienten 100 in der Patientenlagerungsvorrichtung 110 illustriert. Wie die Fig. 1 zeigt, umfasst das Verfahren ein Detektieren 12 einer Aktivität des Patienten basierend auf den Bilddaten und ein Bestimmen 14 von Einordnungsinformation für die Aktivität aus den Bilddaten, wobei die Einordnungsinformation zumindest Information darüber umfasst, ob die detektierte Aktivität des Patienten aktiv vom Patienten oder passiv durch einen äußeren Einfluss hervorgerufen wurde. Das Verfahren 10 umfasst ferner ein Bereitstellen 16 von Information über die Aktivität und der Einordnungsinformation.

In Ausführungsbeispielen wird hier unter der Detektion der Patientenaktivität eine zumindest einfache Bestimmung einer Information darüber verstanden, ob eine Aktivität oder Bewegung vorliegt oder nicht. In einer einfachen Realisierung kann es sich bei dieser Information auch um eine einfache binäre Information handeln. Darüber hinaus kann es sich bei der Einordnungsinformation auch um eine binäre Information handeln die anzeigt, ob eine detektierte Aktivität als passiv oder aktiv eingeordnet oder eingestuft wird. Das Bereitstellen der Information kann insofern auch einer Ausgabe einer binären Information entsprechen.

In einigen Ausführungsbeispielen werden zur Erfassung der Bilddaten in der Umgebung des Patienten 100 und der Patientenlagerungsvorrichtung 110 mehrere Sensoren 140a, 140b verwendet, wie dies in der Fig. 2 illustriert ist. Die Bilddaten können demnach in Ausführungsbeispielen durch ein oder mehrere Bildsensoren oder Kameras erfasst werden. Die Sensoren können dabei zwei- oder mehrdimensional sein und auch Signale im nicht sichtbaren Bereich erfassen, wie z.B. Infrarotsignale, die auch eine entsprechende Verarbeitung von im Dunkeln aufgenommen Bilddaten erlauben. Die Bilddaten selbst können beispielsweise auch Tiefeninformation (dritte Dimension) enthalten, die eine entsprechend verbesserte Verarbeitung bzw. Robustheit der Erkennung und Einordnung der Aktivitäten ermöglichen.

Ein weiteres Ausführungsbeispiel ist ein Computerprogramm mit einem Programmcode zur Durchführung eines der hierin beschriebenen Verfahren, wenn der Programmcode auf einem Computer, einem Prozessor oder einer programmierbaren Hardwarekomponente ausgeführt wird. Ein weiteres Ausführungsbeispiel ist eine Vorrichtung zur Einordnung von Aktivitäten eines Patienten basierend auf Bilddaten des Patienten in einer Patientenlagerungsvorrichtung, mit einer Recheneinrichtung zur Durchführung eines der hierin beschriebenen Verfahren. In Ausführungsbeispielen kann die Recheneinrichtung einem beliebigen Controller oder Prozessor oder einer programmierbaren Hardwarekomponente entsprechen. Beispielsweise kann das Verfahren 10 auch als Software realisiert sein, die für eine entsprechende Hardwarekomponente programmiert ist. Insofern kann die Recheneinrichtung als programmierbare Hardware mit entsprechend angepasster Software implementiert sein. Dabei können beliebige Prozessoren, wie Digitale SignalProzessoren (DSPs) oder Grafikprozessoren zum Einsatz kommen. Ausführungsbeispiele sind dabei nicht auf einen bestimmten Typ von Prozessor eingeschränkt. Es sind beliebige Prozessoren oder auch mehrere Prozessoren zur Implementierung der Recheneinrichtung denkbar.

In einem Ausführungsbeispiel kann eine solche Vorrichtung 1 ... n Sensoren 140a, 140b umfassen, die so ausgerichtet sind, dass sie mindestens den Patienten 100 und seine nähere Umgebung abdecken, wobei n einer ganzen positiven Zahl entspricht. In manchen Ausführungsbeispielen können die Sensoren 140a, 140b im Wesentlichen unabhängig von den für den Menschen sichtbaren Beleuchtungsverhältnissen arbeiten. Die Bilddaten (z.B. Farbbilder oder/und Infrarotbilder oder/und Tiefenbilder) der überwachten Szene werden dann über die ein oder mehreren Sensoren 140a, 140b und über eine entsprechende Infrastruktur (z.B. eine Netzwerk) an eine Recheneinrichtung geliefert.

In Ausführungsbeispielen können verschiedene Typen von Kameras oder Tiefensensoren eingesetzt werden. Die hier vorgestellten Verfahren sind mit unterschiedlichen Kameratypen ausführbar, sowie mit ein oder mehreren Kameras. Einige Ausführungsbeispiele verwenden Tiefenkameras, die ggf. das Definieren der Zonen und die Unterscheidung zwischen intrinsisch/extrinsisch erleichtern. Prinzipiell funktioniert das Verfahren in Ausführungsbeispielen auch mit einem Sensor. Werden mehrere Sensoren verwendet, lassen sich Verschattungseffekte reduzieren und das Verfahren robuster machen. In manchen Ausführungsbeispielen kann ein zusätzlicher Schritt zur extrinsischen Kalibration der Kameras/Sensoren durchgeführt werden.

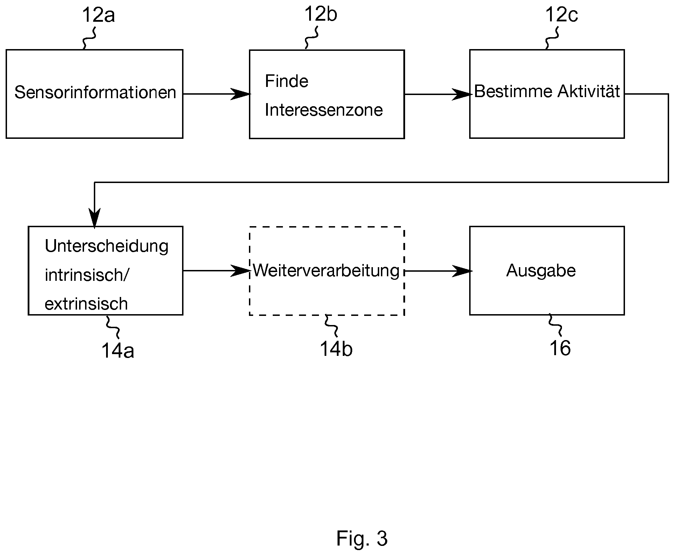

Fig. 3 zeigt ein Flussdiagramm eines weiteren Ausführungsbeispiels. Das Flussdiagramm illustriert einzelne Verfahrensschritte in einem Ausführungsbeispiel, bei dem eine Interessenzone I 120 betrachtet wird. In diesem Ausführungsbeispiel wird eine Interessenzone 120 in den Bilddaten bestimmt, wobei das Detektieren der Aktivität in der Interessenzone 120 erfolgt. In der Fig. 2 ist zusätzlich zu der Interessenzone 120 eine weitere Zone 130 dargestellt. In Ausführungsbeispielen kann eine Bestimmung einer weiteren Zone 130, die die Interessenzone zumindest teilweise umschließt, zusätzlich erfolgen. Wie die Fig. 3 zeigt, erfolgt zunächst eine Erfassung 12a der Sensorinformation in Form der Bilddaten. In den Sensorinformationen wird dann die Interessenzone 120 identifiziert 12b. Dabei kann es sich zum Beispiel um eine Patientenlagerungsvorrichtung 110, den Patienten 100 oder eine bestimmte Körperregion des Patienten 100 handeln. Anschließend wird in der Interessenzone 120 die Aktivität ermittelt, vgl. Schritt 12c. Die Aktivität wird danach hinsichtlich intrinsischer (aktiver) und extrinsischer Aktivität (passiv) unterschieden 14a und die Einordnungsinformation entsprechend bestimmt. Nach einer eventuellen oder optionalen Weiterverarbeitung 14b der bisherigen Ergebnisse erfolgt die Ausgabe 16.

Beispielsweise arbeiten die Sensoren kontaktlos und sind in einigen Metern Entfernung zum Patienten angebracht. In manchen Ausführungsbeispielen ist der Patient daher körperlich durch die Sensorik nicht beeinträchtigt und die Sensorik befindet sich nicht im hygienekritischsten Bereich. In einigen weiteren Ausführungsbeispielen der Vorrichtung ist die Recheneinrichtung als Prozessoreinheit implementiert, die mit den 1... n Sensoren verbunden ist und auf der die beschriebenen Verfahrensschritte ausgeführt werden können. In Ausführungsbeispielen können ferner ein oder mehrere Kommunikationsverbindungen implementiert sein, um die Ausgabe der Recheneinrichtung an abnehmende Systeme weiter zu leiten. Die in der Fig. 3 dargestellten Verfahrensschritte werden im Folgenden näher erläutert.

Als Eingabe erwartet das Verfahren die Sensorinformation oder die Bilddaten der 1..n Sensoren. Je nachdem welche Bilddaten die Sensoren genau liefern, unterscheidet sich die konkrete Ausführung der folgenden Schritte ein wenig. Werden n>1 Sensoren verwendet, können die Eingabedaten der Sensoren räumlich in Zusammenhang gebracht werden, beispielsweise könnte diese zu einer dreidimensionalen Punktewolke zusammengeführt werden, die dann die Bilddaten für die weitere Verarbeitung bildet.

Im zweiten Schritt 12b findet das Verfahren die durch den Benutzer oder ein zweites oder anderes System festgelegte Interessenzone 120. Typische Interessenzonen 120 könnten die Patientenlagerungsvorrichtung (PLV) 110 mit den darauf befindlichen Objekten sein. Auch denkbar wäre eine Einschränkung auf den gesamten Patienten 100 selbst oder auf bestimmte Körperregionen.

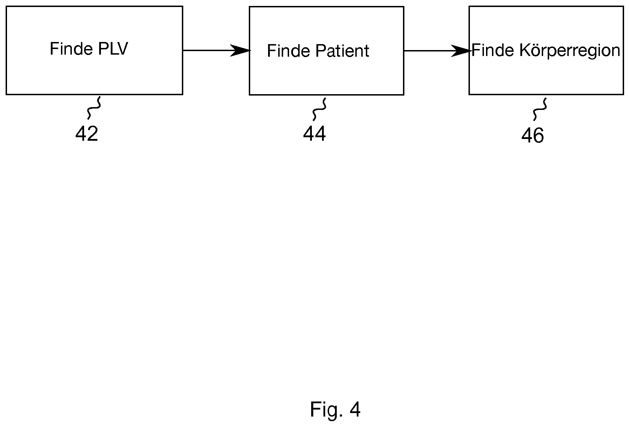

Fig. 4 zeigt ein Blockdiagramm eines weiteren Ausführungsbeispiels eines Verfahrens zur Nachführung einer Interessenzone und fasst die hier aufgeführten Beispiele für eine Interessenzone 120 noch einmal in einem Flussdiagramm zusammen. Demnach kann in den Bilddaten zunächst die Patientenlagerungsvorrichtung 110 gefunden werden, Schritt 42. Im Bereich der Patientenlagerungsvorrichtung 110 kann dann ein Patient 100 identifiziert werden, Schritt 44. Im Bereich des Patienten 100 kann dann in einem nachfolgenden Schritt 46 eine Körperregion gefunden werden. In manchen Ausführungsbeispielen kann eine Objekterkennung des jeweiligen Objekts in der Szene erfolgen. Zur Objekterkennung gibt es eine Reihe von verschiedenen Verfahren, die eingesetzt werden könnten. Stand der Technik sind Verfahren des Deep Learning, wie beispielsweise Girshick et al, siehe oben. In einigen weiteren Ausführungsbeispielen können auch Objektdetektoren verwendet werden, die spezieller auf die Art (bzw. das Objekt) der einzelnen Interessenzone 120 zugeschnitten sind, wie im Folgenden noch näher erläutert werden wird.

Im Schritt 12c in der Fig. 3 wird die Aktivität in der im Schritt 12b festgelegten Interessenzone 120 ermittelt. Dies kann in Ausführungsbeispielen dadurch bewerkstelligt werden, dass Zeitserien der Sensordaten betrachtet werden. Das der Interessenzone 120 entsprechende Teilbild der Sensordaten kann nun für verschiedene Zeitpunkte bestimmt werden (beispielsweise jede 0.1 Sekunden), so dass eine zeitlich sinnvolle Abfolge von Teilbildern entsteht. Durch die Analyse der Veränderungen der Teilbilder über die Zeit, kann im Folgenden die Aktivität quantifiziert werden. Die Analyse der Veränderungen in den n Teilbildern, die in ihrer zeitlichen Abfolge mit t_1... t_n bezeichnet seien, kann auf verschiedene Arten erfolgen.

In Ausführungsbeispielen kommen hierfür verschiedene Möglichkeiten in Frage. Eine Möglichkeit ist die Bestimmung eines absoluten Differenzbildes. Dafür kann beispielsweise für jedes Paar t_i und t_(i-1) die absolute Differenz der Pixelwerte für jede Pixelposition bestimmt werden, was im Ergebnis zu einem resultierenden Differenzbild D führt. Die einzelnen Differenzen können dann mit einem Schwellenwert s_1 verglichen werden und

$$V(x, y) = \begin{cases} 0, wenn\ D(x, y) < s_1 \\ 1, wenn\ D(x, y) \geq s_1 \end{cases}$$

kann entsprechend gesetzt werden. Die Summe der Pixel in V mit Wert 1 kann nun als ein Maß für die Aktivität zum Zeitpunkt i herangezogen werden.

Eine weitere Möglichkeit zur Detektion einer Aktivität ist eine abtastwertbasierte Subtraktion der Hintergrundinformation (auch engl. "sample based background subtraction"). Je nach Wahl der benötigten Schwellwerte im vorangehenden Konzept können die entsprechenden Maße anfällig gegenüber Rauschen sein bzw. nicht sensitiv genug gegenüber echter Bewegung. Zumindest manche Ausführungsbeispiele können daher Methoden verwenden, die die Geschichte eines Pixels ausführlicher berücksichtigen, um zu entscheiden, ob dieser Aktivität repräsentiert oder nicht. Ein bekanntes und erfolgreiches Beispiel hierfür ist ViBe, Barnich, O. et al, siehe oben. ViBe erstellt wiederum eine Aktivitätskarte, in der ein Pixel den Wert 1 erhält, wenn er Aktivität erfährt und den Wert 0, wenn dies nicht der Fall ist. Daher ist auch hier die Summe der Pixelwerte ein Maß für die Aktivität.

Die Kenngrößen für Aktivität können darüber hinaus noch normalisiert werden, indem die Summen durch die Anzahl der Pixel des betrachteten Teilbildes dividiert werden. Die oben beschriebenen Verfahren liefern eine zeitlich sinnvolle Abfolge (Zeitreihe) von Kenngrößen k_1... k_m, die die Aktivität zum Zeitpunkt t_i...t_m beschreiben.

Ausführungsbeispiele unterscheiden ferner zumindest zwischen intrinsischer oder aktiver Aktivität, wenn die Aktivität als vom Patienten selbst hervorgerufen wird, und extrinsischer oder passiver Aktivität, wenn die Aktivität als von außen hervorgerufen eingestuft wird. Wie oben erläutert kann im Schritt 12c die gemessene oder detektierte Aktivität zwar auf eine definierte Zone eingeschränkt werden, dennoch ist dem System bisher der Ursprung der Aktivität nicht bekannt. Es erfolgt daher das Bestimmen 14 der Einordnungsinformation.

In diesem Schritt 14, trifft das Verfahren eine Unterscheidung zwischen intrinsischer und extrinsischer Aktivität. In anderen Ausführungsbeispielen können auch noch andere Einstufung getroffen werden. Im Allgemeinen kann davon ausgegangen werden, dass bei extrinsischer Aktivität auch Aktivität in der näheren Umgebung der betrachteten Interessenzone 120 auftritt. Dieser Gedanke, um potentielle extrinsische Aktivität zu erkennen, wird im vorliegenden Ausführungsbeispiel im Zusammenspiel mit der weiteren Zone 130, vgl. Fig. 2, verfolgt. Weitere, ggfs. differenziertere Lösungsmöglichkeiten, werden weiter unter erläutert.

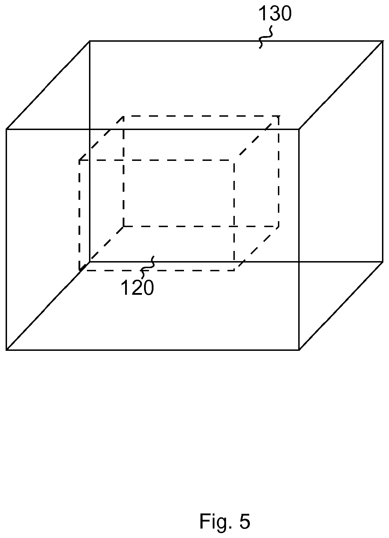

Das Verfahren bestimmt in diesem Ausführungsbeispiel nun um die eigentliche Interessenzone I 120 eine weitere Zone Z 130. Z könnte beispielsweise eine Box sein, die einer in jeder Dimension um einen Wert W vergrößerten Box I entspricht abzüglich der Box I selbst. W könnte dabei auch abhängig sein von der eigentlichen Größe von I. Ein Beispiel für die Zonen I und Z ist in Fig. 5 gezeigt. Fig. 5 zeigt eine Illustration zur Veranschaulichung einer Interessenzone 120, hier als Box dargestellt, und einer weiteren Zone 130, ebenfalls als Box dargestellt, in einem Ausführungsbeispiel.

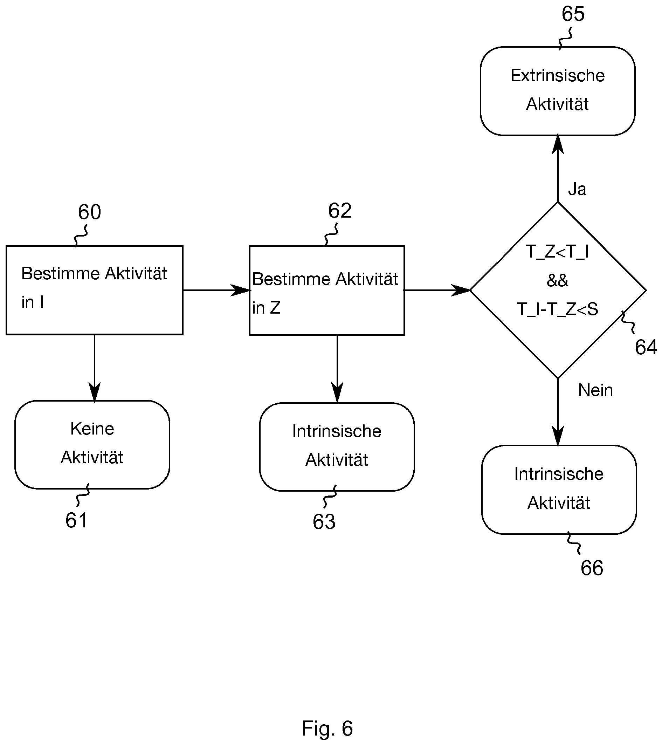

Ist die Zone Z 130 festgelegt, kann die Unterscheidung zwischen extrinsischer und intrinsischer Aktivität in der Interessenzone 120 I erfolgen. Fig. 6 zeigt ein Blockdiagramm eines weiteren Ausführungsbeispiels eines Verfahrens zur Unterscheidung zwischen intrinsischer und extrinsischer Aktivität eines Patienten 100. Wie vorher erläutert, wird zunächst in einem Schritt 60 Aktivität in I ermittelt. Wird hier keine Aktivität festgestellt, ist dies auch die aktuelle Ausgabe des Verfahrens, wie dies im Schritt 61 dargestellt ist. Andernfalls wird die bestimmte Aktivität A_I sowie der dazugehörige Zeitstempel T_I zur Verfügung gestellt. Das Bereitstellen 16 der Information über die Aktivität umfasst im vorliegenden Ausführungsbeispiel auch ein Bereitstellen eines zugehörigen oder zugeordneten Zeitstempels. Das Verfahren fährt dann fort, indem der vorige Schritt "Bestimme Aktivität" für die Zone Z in Schritt 62 wiederholt wird. Wird nun keine Aktivität in Z festgestellt, kann die Aktivität in I als intrinsisch markierte Aktivität im Schritt 63 ausgegeben werden.

Wird eine Aktivität in Z erkannt, so folgt in Schritt 64 ein Vergleich der Zeitstempel, wobei T_Z der zu der detektierten Aktivität in der Zone Z zugehörige Zeitstempel ist. Insofern umfasst das Bestimmen 14 der Einordungsinformation im vorliegenden Ausführungsbeispiel ein Überprüfen von vorhandenen Aktivitäten anhand des Zeitstempels. Erkennt das Verfahren also Aktivität in Z vor einer Aktivität in I (T_Z<T_I in Schritt 64), welche ferner nicht älter als ein Parameter S ist als die Aktivität in I (T_I - T_Z < S in Schritt 64), geht es davon aus, dass es sich um extrinsische Aktivität handelt und gibt diese in Schritt 65 aus. Wird jedoch Aktivität in I vor oder gleichzeitig Aktivität in Z festgestellt, wird diese als intrinsische Aktivität klassifiziert und in Schritt 66 ausgegeben. In manchen Ausführungsbeispielen findet demnach eine Bestimmung, ob eine Aktivität innerhalb der Interessenzone I 120 zu einer Aktivität in der weiteren Zone Z 130 korrespondiert, statt. Die detektierte Aktivität des Patienten 100 kann dann als aktiv vom Patienten 100 hervorgerufen eingeordnet werden, wenn die detektierte Aktivität innerhalb der Interessenzone 120 zu keiner Aktivität in der weiteren Zone 130 korrespondiert. Analog kann die detektierte Aktivität des Patienten 100 als passiv durch äußeren Einfluss hervorgerufen eingeordnet werden, wenn die detektierte Aktivität innerhalb der Interessenzone I 120 zu einer Aktivität in der weiteren Zone Z 130 korrespondiert.

In anderen Worten wird die Aktivität in der Interessenzone I 120 als aktiv vom Patienten 100 hervorrufen eingeordnet, wenn keine (zugehörige) Aktivität in der weiteren Zone Z 130 detektiert wird. Die Aktivität in der Interessenzone I 120 wird als passiv von außen hervorgerufen eigeordnet, wenn eine (zugehörige) Aktivität in der weiteren Zone innerhalb eines Zeitraumes S vor oder gleichzeitig mit der Aktivität in der Interessenzone I 120 detektiert wird. Eine Weiterverarbeitung der bisher gewonnenen Daten, also der Information über die Aktivität und der Einordnungsinformation, ist optional und weiter unten näher erläutert. Das Verfahren liefert in der Ausgabe oder der Bereitstellung 16 die in den vorigen Schritten gewonnenen Daten, beispielsweise an ein abnehmendes System. Dies kann z.B. über eine Kommunikationsverbindung, beispielsweise Ethernet, erfolgen.

In einigen weiteren Ausführungsbeispielen kann das oben beschriebene Verfahren robuster gegenüber Störungen gemacht werden, beispielsweise durch eine automatische Verfolgung der Interessenszone I 120 (engl. Tracking) und durch den bereits oben beschriebenen Einsatz von mehreren Sensoren. In manchen Ausführungsbeispielen kann die Interessenzone I 120 automatisch in einer Szene gefunden und verfolgt werden. In diesem Ausführungsbeispiel umfasst das Verfahren 10 ferner ein Nachführen der Interessenzone 120 basierend auf den Bilddaten. Somit kann in solchen Ausführungsbeispielen eine Benutzerinteraktion reduziert oder vermieden werden, wenn sich die Interessenzone I 120 in der Szene verschiebt. Beispielsweise können auch speziell zugeschnittene Detektoren für verschiedene Interessenzonen verwendet werden. Zur Erkennung einer Patientenlagerungsvorrichtung 110 mit Mitteln der Bildverarbeitung wird beispielsweise auf das Dokument DE 10 2017 006529.2 verwiesen, das ein diesbezügliches Konzept offenbart. Um den Patienten 100 an sich, oder einzelne Körperteile des Patienten 100, zu bestimmen, wird beispielsweise auf Achilles et al, siehe oben, verwiesen.

Die Unterscheidung zwischen intrinsischer und extrinsischer Aktivität lässt sich in weiteren Ausführungsbeispielen verbessern, beispielsweise kann auch die Ursache einer extrinsischen Aktivität ermittelt werden. Dabei können "Aktivierende Objekte", z.B. Personen oder Geräte, erkannt werden, die in die Umgebung der eigentlichen Interessenzone eingebracht werden. In manchen Ausführungsbeispielen umfasst das Verfahren 10 eine Detektion von zumindest einer weiteren Person in der Umgebung des Patienten mittels der Bilddaten und eine Detektion von Interaktionen zwischen der zumindest einen weiteren Person und dem Patienten 100. Ferner kann dann eine Bestimmung der Einordnungsinformation basierend auf der Interaktion erfolgen. Es können in den betrachteten Szenarien Manipulationen weiterer Personen vorkommen. Oftmals kommt eine extrinsische Bewegung oder Aktion des Patienten 100 durch Manipulation durch eine weitere Person. Diese Manipulation kann erkannt werden, vgl. DE102017006529.2

In weiteren Ausführungsbeispielen kann eine Bewegung des Bettes erkannt werden, beispielsweise kann die Patientenlagerungsvorrichtung 110 verstellt werden. Dies kann zumindest in manchen Ausführungsbeispielen unter Umständen auch aus der Ferne, ohne in der Nähe anwesende Personen, geschehen. Ob dies der Fall ist, ließe sich mit einem Verfahren aus den Dokumenten DE102015013031 oder DE3436444 feststellen. Dort wird ein Verfahren beschrieben, mit dem sich die Konfiguration einer PLV bestimmen lässt. Stellt das Verfahren eine Änderung der Konfiguration in der betreffenden Zeitspanne fest, kann Aktivität in dieser Zeitspanne als extrinsisch (durch Änderung der PLV-Konfiguration) markiert oder detektiert werden. Das Verfahren 10 kann dann auch eine Detektion einer Konfigurationsveränderung der Patientenlagerungsvorrichtung basierend auf den Bilddaten umfassen, und das Bestimmen der Einordnungsinformation basierend auf der Konfigurationsveränderung .

Generell können in Ausführungsbeispielen auch mehrere Interessenzonen mit zugehörigen weiteren Zonen definiert werden. Da das Verfahren basierend auf Bilddaten agiert, können parallel oder auch seriell mehrere Paarungen aus Interessen- und weiteren Zonen ausgewertet werden. So könnten beispielsweise auch Körperhälften eines Patienten 100 getrennt überwacht werden, um so Hemiplegia und Paraplegia zu erkennen.

Mobilisierungsgeräte, wie Bettfahrräder und Mobilisierungsschienen, sollen bei der Mobilisierung von Patienten helfen. Mit Hilfe von Verfahren der Objekterkennung und des Trackings lassen sich auch Mobilisierungsgeräte erkennen, die in die Umgebung der Interessenzone I 120 eingebracht werden. Je nach Gerät, kann dieses mit einem Motor versehen sein oder nicht. Im ersten Fall kann damit die Bewegung des Patienten 100 unterstützt oder auch vollkommen übernommen werden. Im zweiten Fall dient es zumindest der Animation zur Bewegung. In jedem Fall aber ist die Bewegung nicht allein intrinsisch motiviert, so dass vorzugsweise eine gesonderte Markierung der Aktivität für den Zeitraum, in dem ein Mobilisierungsgerät in der Interessenzone I erkannt wurde, durch das Verfahren vorgenommen werden kann. Ausführungsbeispiele können daher eine Detektion eines Mobilisierungsgerätes basierend auf den Bilddaten, und ein Bestimmen 14 der Einordungsinformation basierend auf dem Vorhandensein eines Mobilisierungsgerätes vorsehen.

Um die Art des Mobilisierungsgerätes unterscheiden zu können, kann auch eine Aktivität des Patienten aus der Vergangenheit mit einbezogen werden. Dazu können manche Ausführungsbeispiele eine Historie der Aktivitäten eines Patienten erfassen und speichern. Hatte beispielsweise ein Patient 100 gerade eine hohe intrinsische Aktivität, ist es wahrscheinlich, dass das Mobilisierungsgerät nur der Unterstützung dienen soll. Hatte der Patient keine intrinsische Aktivität, ist ein vollunterstützendes System sehr wahrscheinlich.

Darüber hinaus können manche Ausführungsbeispiele auch ein oder mehrere Aktivitätenprofile bestimmen, die Informationen über zeitliche Verläufe von aktiv oder passiv hervorgerufenen Aktivitäten des Patienten umfassen. Insofern kann das Verfahren dahingehend verfeinert werden, indem die gewonnenen Informationen zur intrinsischen und extrinsischen Aktivität weiterverarbeitet werden. Dies könnte entweder direkt auf der Prozessoreinheit/Recheneinrichtung der beschriebenen Vorrichtung geschehen, und somit auch vor der Ausgabe (an weitere Systeme), oder aber womöglich auch in einem anderen System, das die bisherigen Daten empfängt. Eine Möglichkeit der Weiterverarbeitung wäre das Entfernen von extrinsischer Aktivität. Dies könnte dadurch geschehen, dass Bereiche mit extrinsischer Aktivität einfach ersatzlos aus den Aktivitätsdaten entfernt werden. Wird die Aktivität nun über ein Zeitfenster gemittelt, erhält man ein Maß für intrinsische Aktivität des Objektes, beispielsweise des Patienten.

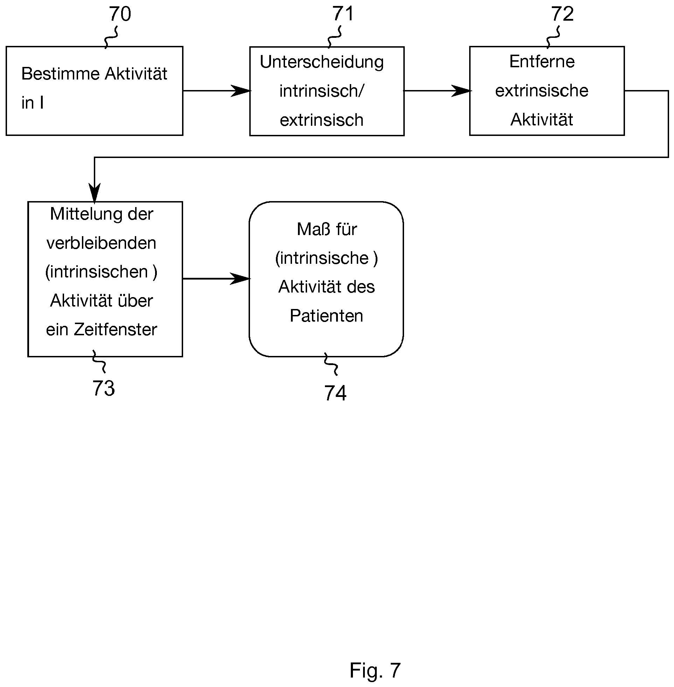

Fig. 7 zeigt eine Veranschaulichung zur Weiterverarbeitung von als intrinsisch und extrinsisch markierten Aktivitäten in einem Ausführungsbeispiel. Das gezeigte Flussdiagramm illustriert eine Weiterverarbeitung der als intrinsisch und extrinsisch markierten Aktivitäten, so dass am Ende des Verfahrens ein Maß für die intrinsische Aktivität des Patienten zur Verfügung steht. Zunächst wird in Schritt 70 eine Aktivität in der Interessenzone detektiert und anschließend in Schritt 71 nach intrinsischer und extrinsischer Aktivität unterschieden. In dem gezeigten Ausführungsbeispiel wird in Schritt 72 die als extrinsisch eingestufte Aktivität entfernt und in Schritt 73 die verbleibende intrinsische Aktivität über ein Zeitfenster gemittelt. Im Ergebnis kann dann ein Maß für die intrinsische Aktivität des Patienten ausgegeben werden.

Andersherum können in anderen Ausführungsbeispielen auch im dritten Schritt 72 des Flussdiagramms Zeitperioden mit intrinsischer Aktivität entfernt werden, wodurch ein Maß für die Aktivität von außen (extrinsische) erhalten werden kann. Auch könnte das Signal geglättet werden, um Messfehler auszugleichen. Dies wäre beispielsweise mit einem "moving average" Filter zu bewerkstelligen. Ähnlich ließe sich ein Mittel über eine längere Zeitspanne bestimmen und als Ausgabe verwenden.

Die Daten könnten auch mit einem Schwellenwert verglichen werden und bei Über- oder Unterschreitung des Schwellwertes könnte ein Event als Ausgabe dienen.

Weiterhin ließen sich die bis zum aktuellen Zeitpunkt X erhobenen Daten extrapolieren. Somit könnte beispielsweise eine Überschreitung eines Schwellenwertes vorausgesagt werden, bevor diese überhaupt aufgetreten ist. Auch ließe sich eine Frequenzanalyse auf dem Aktivitätssignal durchführen, um herauszufinden, ob die Bewegung mit einer oder mehreren prominenten Frequenzen auftritt und welche dies sind.

In weiteren Ausführungsbeispielen könnte die bisher beschriebene Vorrichtung mit dem beschriebenen Verfahren, bzw. das Verfahren selbst, eine Reihe verschiedener Ausgaben tätigen, die für verschiedene Anwendungsfälle sinnvoll sein können. Z.B. kann der errechnete Aktivitätswert als kontinuierliche Messgröße ausgegeben werden. Damit könnte dieser als zusätzlicher Vitalparameter für einen Patienten verfügbar gemacht werden (insbesondere, wenn die extrinsische Aktivität entfernt wird). Auch wäre es denkbar, nur die extrinsische Aktivität zur Verfügung zu stellen, so könnte überwacht werden, wie oft und lang ein Patient in gewissen Zeitspannen gestört wurde (vgl. DE102017006529.2).

Der intrinsische Aktivitätswert (insbesondere in Kombination mit einem Schwellwertvergleich) kann verwendet werden, um bei Schwellenwertüberschreitung Aufwachsituationen zu detektieren. Dies ist beispielsweise dann sinnvoll, wenn ein Patient aus einem Operationssaal kommt und auf sein Erwachen gewartet werden muss. Rhythmische Bewegungen können in manchen Ausführungsbeispielen ebenfalls erkannt werden. Ergibt eine Frequenzanalyse beispielsweise, dass die Bewegung periodischen Änderungen unterliegt, kann die ermittelte Frequenz verwendet werden, um (tonischklonische) Krampfanfälle zu erkennen (vgl. WO02082999). Wird eine einmalige, starke Bewegung festgestellt, besteht die Möglichkeit, dass ein gelagertes Körperteil herabgefallen ist, was somit in manchen Ausführungsbeispielen indiziert werden kann. Definiert man als Interessenzone eine Körperhälfte entlang der Sagitalebene oder entlang der Transversalebene, lässt sich in einigen Ausführungsbeispielen in Verbindung mit der intrinsischen Aktivität im ersteren Fall die Entwicklung einer Hemiplegia und im letzteren Fall einer Paraplegia verfolgen. Ferner könnte in weiteren Ausführungsbeispielen als Interessenzone ein Körperteil oder eine Körperregion definiert werden und die Genesung/Entwicklung derselben entsprechend verfolgt werden.

Generell können Ausführungsbeispiele mit Hilfe von Kameras erfasste Bilddaten von Patienten auswerten und die Patienten so beobachtet werden. Mit Hilfe von erkannter AktivitätBewegung können Rückschlüsse auf den Patientenzustand (Krampfanfall) gezogen werden. Dabei kann zwischen aktiver und passiver Aktivität unterschieden werden und somit von außen induzierte Aktivität detektiert und unterschieden werden. Dabei sind Ausführungsbeispiele nicht auf die Detektion bestimmter Aktivitäten wie Krampfanfälle beschränkt und somit für das Erkennen anders gearteter Aktivität ebenfalls geeignet. Darüber hinaus können manche Ausführungsbeispiele eine Interessenzone adaptiv oder automatisch verfolgen und so beispielsweise die Genesung eines Körperteils beobachtet werden. Ausführungsbeispiele sind daher gegenüber Verschattungen und sich bewegenden Interessenzonen robust. Ausführungsbeispiele kommen dabei ohne an dem Patienten direkt befestigte Sensoren aus. Insbesondere kann so der Fokus auf besondere, sich ggfs. über die Zeit verändernde, Interessenzonen gelegt werden.

In einem weiteren Ausführungsbeispiel wird ein Modell des "Deep Learning" eingesetzt, um die Bilddaten zu verarbeiten. Beispielsweise schließt das Modell direkt aus einer Bildsequenz als Eingabe, ob eine Aktivität des Patienten gerade intrinsisch oder extrinsisch ist. In einem solchen Ausführungsbeispiel würde ein Algorithmus oder ein Verfahren implizit lernen, dass z.B. eine Manipulation des Patienten durch eine weitere Person extrinsische Aktivität verursacht. Einer Eingabe einer Bildsequenz würde sich beispielsweise eine Videoabschnittsklassifikation durch ein tiefes neuronales Netz anschließen, um die Einordnungsinformation zu bestimmen.

Die in der vorstehenden Beschreibung, den Ansprüchen und den Zeichnungen offenbarten Merkmale können sowohl einzeln als auch in beliebiger Kombination für die Verwirklichung von Ausführungsbeispielen in ihren verschiedenen Ausgestaltungen von Bedeutung sein und - soweit sich nicht aus der Beschreibung etwas anderes ergibt - beliebig miteinander kombiniert werden.

Obwohl manche Aspekte im Zusammenhang mit einem Verfahren bzw. einer Vorrichtung beschrieben wurden, versteht es sich, dass diese Aspekte auch eine Beschreibung der entsprechenden Vorrichtung bzw. des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist, und umgekehrt. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar.

Je nach bestimmten Implementierungsanforderungen können Ausführungsbeispiele der Erfindung in Hardware oder in Software implementiert sein. Die Implementierung kann unter Verwendung eines digitalen Speichermediums, beispielsweise einer Floppy-Disk, einer DVD, einer Blu-Ray Disc, einer CD, eines ROM, eines PROM, eines EPROM, eines EEPROM oder eines FLASH-Speichers, einer Festplatte oder eines anderen magnetischen oder optischen Speichers durchgeführt werden, auf dem elektronisch lesbare Steuersignale gespeichert sind, die mit einer programmierbaren Hardwarekomponente derart zusammenwirken können oder zusammenwirken, dass das jeweilige Verfahren durchgeführt wird.

Eine programmierbare Hardwarekomponente kann durch einen Prozessor, einen Computerprozessor (CPU = Central Processing Unit), einen Grafikprozessor (GPU = Graphics Processing Unit), einen Computer, ein Computersystem, einen anwendungsspezifischen integrierten Schaltkreis (ASIC = Application-Specific Integrated Circuit), einen integrierten Schaltkreis (IC = Integrated Circuit), ein Ein-Chip-System (SOC = System on Chip), ein programmierbares Logikelement oder ein feldprogrammierbares Gatterarray mit einem Mikro-prozessor (FPGA = Field Programmable Gate Array) gebildet sein.

Das digitale Speichermedium kann daher maschinen- oder computerlesbar sein. Manche Ausführungsbeispiele umfassen also einen Datenträger, der elektronisch lesbare Steuersignale aufweist, die in der Lage sind, mit einem programmierbaren Computersystem oder einer programmierbaren Hardwarekomponente derart zusammenzuwirken, dass eines der hierin beschriebenen Verfahren durchgeführt wird. Ein Ausführungsbeispiel ist somit ein Datenträger (oder ein digitales Speichermedium oder ein computerlesbares Medium), auf dem das Programm zum Durchführen eines der hierin beschriebenen Verfahren aufgezeichnet ist.

Allgemein können Ausführungsbeispiele der vorliegenden Erfindung als Programm, Firmware, Computerprogramm oder Computerprogrammprodukt mit einem Programmcode oder als Daten implementiert sein, wobei der Programmcode oder die Daten dahin gehend wirksam ist bzw. sind, eines der Verfahren durchzuführen, wenn das Programm auf einem Prozessor oder einer programmierbaren Hardwarekomponente abläuft. Der Programmcode oder die Daten kann bzw. können beispielsweise auch auf einem maschinenlesbaren Träger oder Datenträger gespeichert sein. Der Programmcode oder die Daten können unter anderem als Quellcode, Maschinencode oder Bytecode sowie als anderer Zwischencode vorliegen.

Ein weiteres Ausführungsbeispiel ist ferner ein Datenstrom, eine Signalfolge oder eine Sequenz von Signalen, der bzw. die das Programm zum Durchführen eines der hierin beschriebenen Verfahren darstellt bzw. darstellen. Der Datenstrom, die Signalfolge oder die Sequenz von Signalen kann bzw. können beispielsweise dahin gehend konfiguriert sein, um über eine Datenkommunikationsverbindung, beispielsweise über das Internet oder ein anderes Netzwerk, transferiert zu werden. Ausführungsbeispiele sind so auch Daten repräsentierende Signalfolgen, die für eine Übersendung über ein Netzwerk oder eine Datenkommunikationsverbindung geeignet sind, wobei die Daten das Programm darstellen.

Ein Programm gemäß einem Ausführungsbeispiel kann eines der Verfahren während seiner Durchführung beispielsweise dadurch umsetzen, dass dieses Speicherstellen ausliest oder in diese ein Datum oder mehrere Daten hinein schreibt, wodurch gegebenenfalls Schaltvorgänge oder andere Vorgänge in Transistorstrukturen, in Verstärkerstrukturen oder in anderen elektrischen, optischen, magnetischen oder nach einem anderen Funktionsprinzip arbeitenden Bauteile hervorgerufen werden. Entsprechend können durch ein Auslesen einer Speicherstelle Daten, Werte, Sensorwerte oder andere Informationen von einem Programm erfasst, bestimmt oder gemessen werden. Ein Programm kann daher durch ein Auslesen von einer oder mehreren Speicherstellen Größen, Werte, Messgrößen und andere Informationen erfassen, bestimmen oder messen, sowie durch ein Schreiben in eine oder mehrere Speicherstellen eine Aktion bewirken, veranlassen oder durchführen sowie andere Geräte, Maschinen und Komponenten ansteuern.

**Patentansprüche**

1. Computerimplementiertes Verfahren (10) zur Einordnung von Aktivitäten eines Patienten (100) basierend auf Bilddaten des Patienten (100) in einer Patientenlagerungsvorrichtung (110), mit

    - Detektieren (12) einer Aktivität des Patienten (100) basierend auf den Bilddaten;
    **gekennzeichnet durch**
    - Bestimmen einer Interessenzone (120) in den Bilddaten, wobei das Detektieren der Aktivität in der Interessenzone (120) erfolgt;
    - Bestimmen einer weiteren Zone (130), die die Interessenzone (120) zumindest teilweise umschließt;
    - Bestimmen (14) von Einordnungsinformation für die Aktivität aus den Bilddaten, wobei die Einordnungsinformation zumindest Information darüber umfasst, ob die detektierte Aktivität des Patienten (100) aktiv vom Patienten (100) oder passiv durch einen äußeren Einfluss hervorgerufen wurde,

        wobei die Aktivität in der Interessenszone (120) als aktiv vom Patienten (100) hervorgerufen eingeordnet wird, wenn keine Aktivität in der weiteren Zone (130) detektiert wird, und
        wobei die Aktivität in der Interessenszone (120) als passiv von außen hervorgerufen eingeordnet wird, wenn eine Aktivität in der weiteren Zone (130) innerhalb eines Zeitraums vor oder gleichzeitig mit der Aktivität in der Interessenszone (120) detektiert wird; und

    - Bereitstellen (16) von Information über die Aktivität und der Einordnungsinformation.

2. Verfahren (10) gemäß Anspruch 1, ferner mit

    - Erfassen der Bilddaten in der Umgebung des Patienten (100) und der Patientenlagerungsvorrichtung (110) mit mehreren Sensoren (140a; 140b).

3. Verfahren (10) gemäß einem der vorangehenden Ansprüche, wobei das Bereitstellen (16) der Information über die Aktivität einen zugeordneten Zeitstempel umfasst.

4. Verfahren (10) gemäß Anspruch 3, wobei das Bestimmen (14) der Einordungsinformation ein Überprüfen von vorhandenen Aktivitäten anhand des Zeitstempels umfasst.

5. Verfahren (10) gemäß einem der Ansprüche 1 bis 4, das ferner ein Nachführen der Interessenzone (120) basierend auf den Bilddaten umfasst.

6. Verfahren (10) gemäß einem der vorangehenden Ansprüche, mit

    - Detektieren von zumindest einer weiteren Person in der Umgebung des Patienten (100) mittels der Bilddaten,
    - Detektieren von Interaktionen zwischen der zumindest einen weiteren Person und dem Patienten (100), und
    - Bestimmen der Einordnungsinformation basierend auf der Interaktion.

7. Verfahren (10) gemäß einem der vorangehenden Ansprüche, mit

    - Detektieren einer Konfigurationsveränderung der Patientenlagerungsvorrichtung (110) basierend auf den Bilddaten, und
    - Bestimmen der Einordnungsinformation basierend auf der Konfigurationsveränderung.

8. Verfahren (10) gemäß einem der vorangehenden Ansprüche, mit

    - Detektieren eines Mobilisierungsgerätes basierend auf den Bilddaten, und
    - Bestimmen der Einordungsinformation basierend auf dem Vorhandensein eines Mobilisierungsgerätes.

9. Verfahren (10) gemäß einem der vorangehenden Ansprüche, mit

    - Bestimmen eines Aktivitätenprofils, das Information über einen zeitlichen Verlauf von aktiv oder passiv hervorgerufenen Aktivitäten des Patienten (100) umfasst.

10. Vorrichtung (10) mit einer Recheneinrichtung, die ausgebildet ist, um ein Verfahren gemäß einem der vorangehenden Ansprüche durchzuführen.

11. Computerprogramm mit einem Programmcode zur Durchführung eines der Verfahren gemäß einem der Ansprüche 1 bis 9, wenn der Programmcode auf einem Computer, einem Prozessor oder einer programmierbaren Hardwarekomponente ausgeführt wird.

**Claims**

1. Computer-implemented method (10) for classifying activities of a patient (100) on the basis of image data of the patient (100) in a patient-supporting apparatus (110), including

    - detecting (12) an activity of the patient (100) on the basis of the image data;
    **characterized by**
    - determining a zone of interest (120) in the image data, the activity being detected within the zone of interest (120);
    - determining a further zone (130) which at least partially encloses the zone of interest (120);
    - determining (14) classification information for the activity from the image data, the classification information at least comprising information as to whether the detected activity of the patient (100) was brought about actively by the patient (100) or passively by an external influence,

        the activity in the zone of interest (120) being classified as actively brought about by the patient (100) if no activity is detected in the further zone (130), and
        the activity in the zone of interest (120) being classified as passively brought about externally if an activity is detected in the further zone (130) within a time period before, or at the same time as, the activity in the zone of interest (120); and

    - providing (16) information about the activity and the classification information.

2. Method (10) according to Claim 1, further including

    - capturing the image data in the surroundings of the patient (100) and the patient-supporting apparatus (110) using a plurality of sensors (140a; 140b).

3. Method (10) according to any of the preceding claims, wherein the provision (16) of the information about the activity comprises an associated timestamp.

4. Method (10) according to Claim 3, wherein the determination (14) of the classification information comprises a verification of activities present on the basis of the timestamp.

5. Method (10) according to any of Claims 1 to 4, further comprising a tracking of the zone of interest (120) on the basis of the image data.

6. Method (10) according to any of the preceding claims, including

    - detecting at least one further person in the surroundings of the patient (100) by means of the image data,
    - detecting interactions between the at least one further person and the patient (100), and
    - determining the classification information on the basis of the interaction.

7. Method (10) according to any of the preceding claims, including

    - detecting a change in configuration of the patient-supporting apparatus (110) on the basis of the image data and
    - determining the classification information on the basis of the change in configuration.

8. Method (10) according to any of the preceding claims, including

    - detecting a mobilization device on the basis of the image data and
    - determining the classification information on the basis of the presence of a mobilization device.

9. Method (10) according to any of the preceding claims, including

    - determining an activity profile which comprises information about a chronological sequence of actively or passively brought about activities of the patient (100).

10. Apparatus (10) having a computing device configured to carry out a method according to any of the preceding claims.

11. Computer program having program code for carrying out one of the methods according to any of Claims 1 to 9 when the program code is carried out on a computer, a processor or a programmable hardware component.

## Revendications

1. Procédé (10) mis en oeuvre par ordinateur et permettant de classer les activités d'un patient (100) en se basant sur des données d'image du patient (100) au sein d'un dispositif de positionnement de patient (110), comprenant une étape consistant à :

   détecter (12) une activité du patient (100) en se basant sur les données d'image ;
   **caractérisé par** les étapes consistant à :

   - déterminer une zone d'intérêt (120) dans les données d'image, la détection de l'activité se faisant dans la zone d'intérêt (120) ;
   - déterminer une zone supplémentaire (130) entourant au moins partiellement la zone d'intérêt (120) ;
   - déterminer (14) des informations de classement concernant l'activité en se basant sur les données d'image, les informations de classement comprenant au moins des informations indiquant si l'activité détectée du patient (100) a été provoquée de manière active par le patient (100) ou de manière passive par une influence extérieure,

   l'activité dans la zone d'intérêt (120) étant classée comme provoquée de manière active par le patient (100) si aucune activité n'est détectée dans la zone supplémentaire (130), et
   l'activité dans la zone d'intérêt (120) étant classée comme provoquée de manière passive par l'extérieur si une activité est détectée dans la zone supplémentaire (130) pendant une période préalable ou concomitante à l'activité dans la zone d'intérêt (120) ; et

   - fournir (16) des informations sur l'activité et les informations de classement.

2. Procédé (10) selon la revendication 1, comprenant en outre l'étape consistant à :

   - enregistrer les données d'image dans l'environnement du patient (100) et du dispositif de positionnement de patient (110) grâce à plusieurs capteurs (140a ; 140b).

3. Procédé (10) selon l'une quelconque des revendications précédentes, dans lequel la fourniture (16) des informations sur l'activité comprend un horodatage associé.

4. Procédé (10) selon la revendication 3, dans lequel la détermination (14) des informations de classement comprend une vérification des activités existantes grâce à l'horodatage.

5. Procédé (10) selon l'une quelconque des revendications 1 à 4, comprenant en outre une mise à jour de la zone d'intérêt (120) en se basant sur les données d'image.

6. Procédé (10) selon l'une quelconque des revendications précédentes, comprenant les étapes consistant à :

   - détecter au moins une personne supplémentaire dans l'environnement du patient (100) à l'aide des données d'image,
   - détecter des interactions entre la au moins une personne supplémentaire et le patient (100), et
   - déterminer les informations de classement en se basant sur l'interaction.

7. Procédé (10) selon l'une quelconque des revendications précédentes, comprenant les étapes consistant à :

   - détecter un changement de configuration du dispositif de positionnement de patient (110) en se basant sur les données d'image, et
   - déterminer les informations de classement en se basant sur le changement de configuration.

8. Procédé (10) selon l'une quelconque des revendications précédentes, comprenant les étapes consistant à :

   - détecter un appareil de mobilisation en se basant sur les données d'image, et
   - déterminer les informations de classement en se basant sur la présence d'un appareil de mobilisation.

9. Procédé (10) selon l'une quelconque des revendications précédentes, comprenant une étape consistant à :

   - déterminer un profil d'activité qui comprend des informations sur un déroulement temporel d'activités du patient (100) provoquées de manière active ou passive.

10. Dispositif (10) comprenant un dispositif de calcul conçu pour mettre en oeuvre un procédé selon l'une quelconque des revendications précédentes.

11. Programme informatique comprenant un code de programme permettant de mettre en oeuvre un des procédés selon l'une quelconque des revendications 1 à 9 lorsque le code de programme est exécuté sur un ordinateur, un processeur ou un composant matériel programmable.

Detektieren einer Aktivität des Patienten basierend auf Bilddaten

12

Bestimmen von Einordnungsinformation
für die Aktivität aus den Bilddaten

14

Bereitstellen von Information über die
Aktivität und der Einordnungsinformation

16

10

Fig. 1

Fig. 2

Fig. 3

| Finde PLV | | Finde Patient | | Finde Körperregion |
|:---:|:---:|:---:|:---:|:---:|

42          44          46

Fig. 4

130

120

Fig. 5

65

Extrinsische
Aktivität

60

Bestimme Aktivität
in I

62

Bestimme Aktivität
in Z

Ja

T_Z<T_I
&&
T_I-T_Z<S

64

Keine
Aktivität

61

Intrinsische
Aktivität

63

Nein

Intrinsische
Aktivität

66

Fig. 6

70

Bestimme Aktivität
in I

71

Unterscheidung
intrinsisch/
extrinsisch

72

Entferne
extrinsische
Aktivität

73

Mittelung der
verbleibenden
(intrinsischen )
Aktivität über
ein Zeitfenster

74

Maß für
(intrinsische )
Aktivität des
Patienten

Fig. 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 02082999 A
- DE 102013017264 A1
- DE 102017006529
- DE 102015013031
- DE 3436444

**In der Beschreibung aufgeführte Nicht-Patentliteratur**

- **KARANTONIS et al.** *Implementation of a Real-Time Human Movement Classifier Using a Triaxial Accelerometer for Ambulatory Monitoring,* 2006
- **ACHILLES, F.** Human Pose Estimation Under Blanket Occlusion for Hospital Monitoring Applications. *International Conference on Medical Image Computing and Computer-Assisted Intervention,* 2016
- **BARNICH, O. ; VAN DROOGENBROECK, M.** A universal background subtraction algorithm for video sequences. *Image Processing, IEEE Transactions on,* 2011
- **GIRSHICK, R. ; DONAHUE, J. ; DARRELL, T. ; MALIK, J.** Rich feature hierarchies for accurate object detection and semantic segmentation. *CVPR (computer Vision and Pattern Recognition,* 2014